(19) Europäisches Patentamt
European Patent Office
Office européen des brevets

(11) Veröffentlichungsnummer: **0 519 290 A1**

(12) # EUROPÄISCHE PATENTANMELDUNG

(21) Anmeldenummer: **92109623.6**

(51) Int. Cl.[5]: **C07D 215/38**, A01N 43/42

(22) Anmeldetag: **09.06.92**

(30) Priorität: **21.06.91 DE 4120477**

(43) Veröffentlichungstag der Anmeldung:
**23.12.92 Patentblatt 92/52**

(84) Benannte Vertragsstaaten:
**BE CH DE DK ES FR GB IT LI NL SE**

(71) Anmelder: **BAYER AG**

**W-5090 Leverkusen 1 Bayerwerk(DE)**

(72) Erfinder: **Jeschke, Peter, Dr.**
**Heymannstrasse 38**
**W-5090 Leverkusen 1(DE)**
Erfinder: **Lindner, Werner, Dr.**
**Märchenstrasse 39**
**W-5000 Köln 80(DE)**
Erfinder: **Müller, Nikolaus, Dr.**
**Knipprather Strasse 98**
**W-4019 Monheim(DE)**
Erfinder: **Harder, Achim, Dr.**
**Piccoloministrasse 398**
**W-5000 Köln 80(DE)**
Erfinder: **Mencke, Norbert, Dr.**
**Grunder-Mühle 2**
**W-5090 Leverkusen 3(DE)**

(54) **Verwendung von 6(7)-aminosubstituierten Chinolin-5,8-chinonen zur Bekämpfung von Endoparasiten, neue 6(7)-aminosubstituierte Chinolin-5,8-chinone und Verfahren zu ihrer Herstellung.**

(57) Die vorliegende Erfindung betrifft die Verwendung von 6(7)-aminosubstituierten Chinolin-5,8-chinonen der allgemeinen Formel (I)

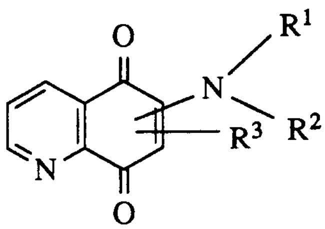

(I),

in welcher

$R^1$ für substituiertes $C_1$-$C_6$-Alkyl, sowie für gegebenenfalls substituiertes Cycloalkylalkyl, Cycloalkyl, Aralkyl oder Aryl steht, wobei als Substituenten genannt seien Halogen, Nitro, Alkyl, Alkylamino, Aminoacyl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl,

$R^2$ für Wasserstoff, Alkyl steht oder $R^1$ und $R^2$ gemeinsam mit dem angrenzenden N-Atom für einen carbocyclischen 5-, 6- oder 7-gliedrigen Ring stehen, der gegebenenfalls auch durch O, N, S unterbrochen sein kann und gegebenenfalls durch $C_{1-4}$-Alkyl substituiert ist,

$R^3$ für Halogen oder -S-$R^4$ steht,

$R^4$ für Alkyl steht,

zur Bekämpfung von Endoparasiten in der Medizin und Tiermedizin, sowie neue 6(7)-aminosubstituierte Chinolin-5,8-chinone und deren Herstellung.

EP 0 519 290 A1

Die vorliegende Erfindung betrifft die Verwendung von 6(7)-aminosubstituierten Chinolin-5,8-chinonen zur Bekämpfung von Endoparasiten, neue 6(7)-aminosubstituierte Chinolin-5,8-chinone und Verfahren zu ihrer Herstellung.

6(7)-Aminosubstituierte Chinolin-5,8-chinone sind bereits bekannt. Es ist jedoch nichts über ihre Verwendung gegen Endoparasiten bekannt (vgl. DE-OS 21 36 037; JP 58 06 76 72; NL 65 05 524).

Die vorliegende Erfindung betrifft:

1. die Verwendung von 6(7)-aminosubstituierten Chinolin-5,8-chinonen der allgemeinen Formel (I)

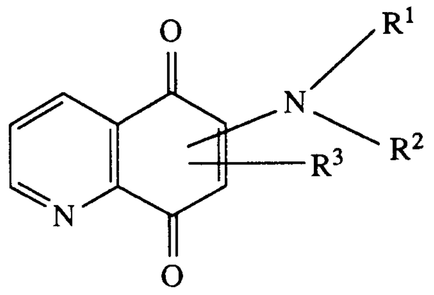

(I),

in welcher

$R^1$ für substituiertes $C_1$-$C_6$-Alkyl, sowie für gegebenenfalls substituiertes Cycloalkylalkyl, Cycloalkyl, Aralkyl oder Aryl steht, wobei als Substituenten genannt seien Halogen, Nitro, Alkyl, Alkylamino, Aminoacyl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl,

$R^2$ für Wasserstoff, Alkyl steht oder $R^1$ und $R^2$ gemeinsam mit dem angrenzenden N-Atom für einen carbocyclischen 5-, 6- oder 7-gliedrigen Ring stehen, der gegebenenfalls auch durch O, N, S unterbrochen sein kann und gegebenenfalls durch $C_{1-4}$-Alkyl substituiert ist,

$R^3$ für Halogen oder -S-$R^4$ steht,

$R^4$ für Alkyl steht,

zur Bekämpfung von Endoparasiten in der Medizin und Tiermedizin.

Die Verbindungen der Formel (I) sind teilweise bekannt und lassen sich analog zu bekannten Verfahren herstellen.

2. Neue 6(7)-aminosubstituierte Chinolin-5,8-chinone der allgemeinen Formel (Ia)

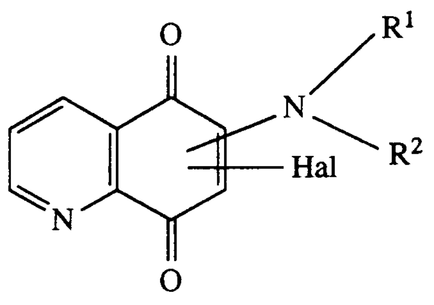

(Ia),

in welcher

$R^1$ für Cycloalkylalkyl oder Aralkyl steht, die gegebenenfalls durch Halogen, Nitro, Alkyl, Alkylamino, Aminoacyl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl substituiert sind,

$R^2$ für Wasserstoff steht,

Hal für Halogen, vorzugsweise Chlor oder Brom, insbesondere Chlor, steht.

3. Verfahren zur Herstellung der neuen 6(7)-aminosubstituierten Chinolin-5,8-chinone der Formel (Ia)

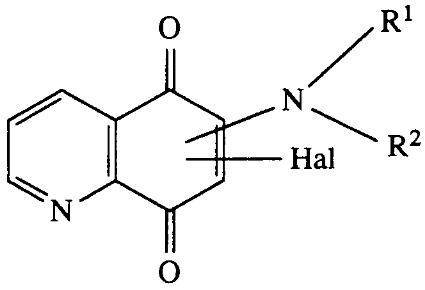

(Ia),

in welcher
$R^1$, $R^2$ und Hal die unter Punkt 2 angegebene Bedeutung besitzen,
dadurch gekennzeichnet, daß man
a) Verbindungen der Formel (II)

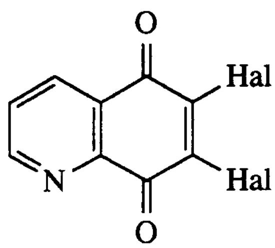

(II),

in welcher
Hal die oben angegebene Bedeutung besitzt,
mit Aminoverbindungen der Formel (III)

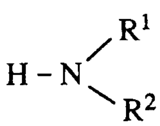

(III),

in welcher
$R^1$ und $R^2$ die oben angegebene Bedeutung haben,
gegebenenfalls in Gegenwart von Metallsalzen und gegebenenfalls in Gegenwart eines Verdünnungsmittels umsetzt, oder indem man
b) Verbindungen der Formel (IV)

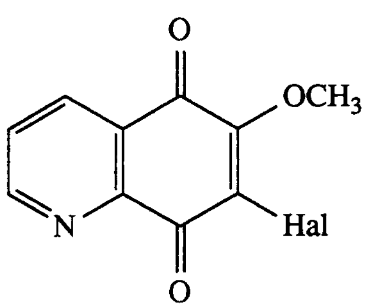

(IV),

in welcher
Hal die oben angegebene Bedeutung besitzt,
mit Aminoverbindungen der Formel (III)

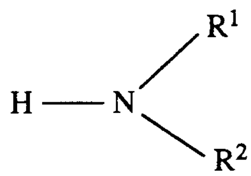

(III),

in welcher

$R^1$ und $R^2$ die oben angegebene Bedeutung haben,

gegebenenfalls in Gegenwart eines Verdünnungsmittels umsetzt.

4. Neue 6(7)-aminosubstituierte Chinolin-5,8-chinone der Formel (Ib)

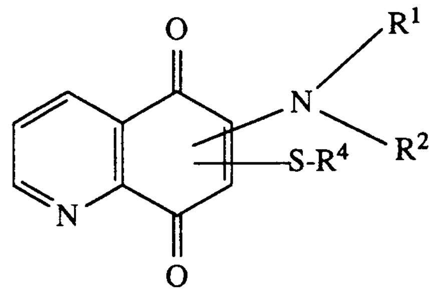

(Ib),

in welcher

$R^1$ für substituiertes $C_1$-$C_6$-Alkyl, sowie für gegebenenfalls substituiertes Cycloalkylalkyl, Cycloalkyl, Aralkyl oder Aryl steht, wobei als Substituenten genannt seien Halogen, Nitro, Alkyl, Alkylamino, Aminoaryl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl,

$R^2$ für Wasserstoff, Alkyl steht oder $R^1$ und $R^2$ gemeinsam mit dem angrenzenden N-Atom für einen carbocyclischen 5-, 6- oder 7-gliedrigen Ring stehen, der gegebenenfalls auch durch O, N, S unterbrochen sein kann und gegebenenfalls durch $C_{1-4}$-Alkyl substituiert ist,

$R^4$ für Alkyl steht.

5. Verfahren zur Herstellung der neuen 6(7)-aminosubstituierten Chinolin-5,8-chinone der Formel (Ib)

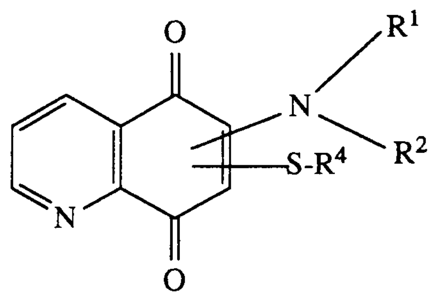

(Ib),

in welcher

$R^1$, $R^2$ und $R^4$ die unter Punkt 4 angegebene Bedeutung besitzen,

dadurch gekennzeichnet, daß man

a) die z.B. gemäß Verfahren 3 erhältlichen 6(7)-aminosubstituierten Chinolin-5,8-chinone der Formel (Ia)

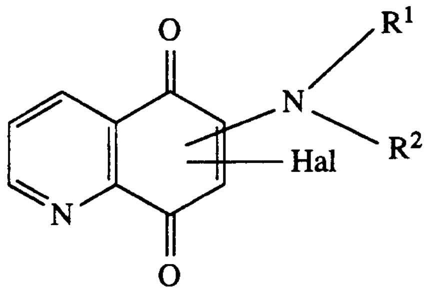

(Ia),

in welcher
$R^1$, $R^2$ und Hal die unter Punkt 3 und 4 angegebene Bedeutung haben,
in einem ersten Reaktionsschritt mit Alkalimetallsulfiden der Formel (V)

$M_2S$ (V),

in welcher
M für ein einwertiges Alkalimetallkation, vorzugsweise Kalium oder Natrium, insbesondere Natrium, steht,
dann in einem zweiten Reaktionsschritt das resultierende Alkalimetallsalz der Formel (VI)

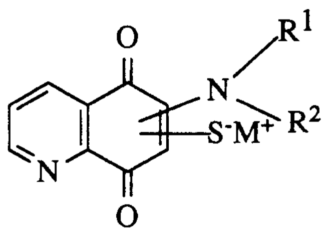

(VI),

in welcher
$R^1$, $R^2$ die oben angegebene Bedeutung besitzen und
M für ein salzartig gebundenes Metallkationenäquivalent steht,
mit Alkylierungsmitteln der Formel (VII)

$R^4$-E (VII),

in welcher
$R^4$ die oben angegebene Bedeutung besitzt und
E für eine elektronenziehende Abgangsgruppe steht,
gegebenenfalls in Gegenwart eines Verdünnungsmittels umsetzt, oder indem man
b) Verbindungen der Formel (Ia)

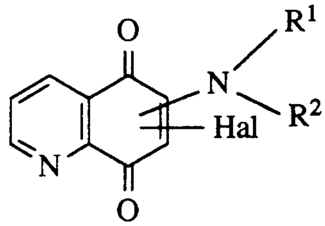

(Ia),

in welcher
$R^1$, $R^2$ und Hal die unter Punkt 3 und 4 angegebene Bedeutung besitzen,
mit Thiolen oder deren Alkalimetallsalzen der Formel (VIII)

$R^4$-$S^-M^+$ (VIII),

in welcher

M für Wasserstoff oder für ein salzartig gebundenes Metallkationenäquivalent steht,

$R^4$ die oben angegebene Bedeutung hat,

in Gegenwart eines Verdünnungsmittels und gegebenenfalls in Gegenwart eines Reaktionshilfsmittels umsetzt.

Die Verbindungen der Formel (I) eignen sich hervorragend zur Bekämpfung von Endoparasiten, besonders auf dem Gebiet der Veterinärmedizin.

Die erfindungsgemäßen 6(7)-aminosubstituierten Chinolin-5,8-chinonderivate sind durch die Formel (I) allgemein definiert.

Bevorzugt sind Verbindungen der Formel (I), in welcher

$R^1$ für substituiertes $C_1$-$C_6$-Alkyl, sowie für gegebenenfalls substituiertes $C_3$-$C_6$-Cycloalkyl-$C_1$-$C_2$-alkyl, $C_3$-$C_6$-Cycloalkyl, Phenyl-$C_1$-$C_2$-alkyl, Naphthyl-$C_1$-$C_2$-alkyl, Phenyl steht, wobei als Substituenten genannt seien eine oder mehrere gleiche oder verschiedene Reste der Gruppe $C_1$-$C_4$-Alkyl, insbesondere Methyl oder Ethyl, $C_1$-$C_4$-Alkoxy, insbesondere Methoxy, Ethoxy, Methylendioxy oder Ethylendioxy, die gegebenenfalls durch Fluor oder Chlor substituiert sind, $C_1$-$C_4$-Halogenalkoxy, insbesondere Trifluormethoxy, Fluorchlorethoxy, $C_1$-$C_4$-Alkylthio, insbesondere Methylthio, $C_1$-$C_4$-Halogenalkylthio, insbesondere Trifluormethylthio, Fluorchlormethylthio, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Halogen, insbesondere Fluor oder Chlor, $NO_2$, CN, Amino, $C_1$-$C_4$-Alkyl- oder Dialkylamino, $C_1$-$C_4$-Halogenalkylamino, Acylamino, insbesondere Acetylamino, Phenyl, Phenylthio, Phenoxy, die gegebenenfalls durch einen der oben angegebenen Reste substituiert sind,

$R^2$ für Wasserstoff, $C_1$-$C_6$-Alkyl steht oder $R^1$ und $R^2$ gemeinsam mit dem angrenzenden N-Atom für einen carbocyclischen 5-, 6- oder 7-gliedrigen Ring stehen, der gegebenenfalls auch durch O, N oder S unterbrochen sein kann und gegebenenfalls durch $C_1$-$C_4$-Alkyl substituiert ist,

$R^3$ für Halogen, vorzugsweise Chlor oder Brom, insbesondere Chlor, oder $C_1$-$C_4$-Alkylthio steht.

Besonders bevorzugt sind Verbindungen der Formel (I)

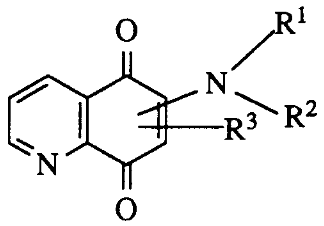

(I),

in welcher

$R^1$ die weiter oben angegebene bevorzugte Bedeutung besitzt,

$R^2$ für Wasserstoff steht,

$R^3$ für Halogen wie Chlor der $C_1$-$C_4$-Alkylthio steht.

Ganz besonders bevorzugt sind Verbindungen der Formel (I), in welcher

$R^1$ für substituiertes $C_1$-$C_6$-Alkyl, sowie für gegebenenfalls substituiertes 1-Cyclohexyl-ethyl, Cyclopropyl, Cyclopentyl, Cyclohexyl, Benzyl, 1-Phenyl-ethyl, 1-Naphthyl-ethyl, Phenyl steht, wobei als Substituenten genannt seien Wasserstoff, Halogen, insbesondere Chlor oder Fluor, $C_1$-$C_4$-Alkyl, insbesondere Methyl oder Ethyl, $C_1$-$C_4$-Halogenalkyl, insbesondere Trifluormethyl, $C_1$-$C_4$-Alkylthio, insbesondere Methylthio, $C_1$-$C_4$-Halogenalkylthio, insbesondere Trifluormethylthio, $C_1$-$C_4$-Alkoxy, insbesondere Methoxy, $C_1$-$C_4$-Halogenalkoxy, insbesondere Trifluormethoxy, Phenyl, $NO_2$,

$R^2$ für Wasserstoff steht,

$R^3$ für Chlor oder $C_1$-$C_4$-Alkylthio, insbesondere Methylthio, Ethylthio oder Propylthio, steht.

Im einzelnen seien folgende Verbindungen der Formel (I) genannt, in welcher die Reste $R^1$, $R^2$, $R^3$ die folgende Bedeutung haben:

(±)-6(7)-Methylthio-7(6)-[1-(4-methoxy-phenyl)-ethylamino]-chinolin-5,8-chinon,

(±)-6(7)-Methylthio-7(6)-[1-(4-methyl-phenyl)-ethylamino]-chinolin-5,8-chinon,

(±)-6(7)-Methylthio-7(6)-[1-(4-chlor-phenyl)-ethylamino]-chinolin-5,8-chinon,
(±)-6(7)-Propylthio-7(6)-[1-(2,4-dichlor-phenyl)-ethylamino]-chinolin-5,8-chinon,
(±)-6(7)-Propylthio-7(6)-[1-(3,4-dichlor-phenyl)ethylamino]-chinolin-5,8-chinon,
(±)-6(7)-Propylthio-7(6)-[1-(4-fluor-phenyl)-ethylamino]-chinolin-5,8-chinon,
R-(+)-7-Propylthio-6-(1-phenyl-ethylamino)-chinolin-5,8-chinon,
S-(-)-7-Propylthio-6-(1-phenyl-ethylamino)-chinolin-5,8-chinon,
R-(+)-7-Methylthio-6-(1-cyclohexyl-ethylamino)-chinolin-5,8-chinon,
S-(-)-7-Methylthio-6-(1-cyclohexyl-ethylamino)-chinolin-5,8-chinon,
7-Methylthio-6-cyclopropylamino-chinolin-5,8-chinon,
7-Propylthio-6-cyclopentylamino-chinolin-5,8-chinon,
7-Ethylthio-6-(4-trifluormethoxy-phenylamino)-chinolin-5,8-chinon,
7-Ethylthio-6-(4-trifluormethylthio-phenylamino)-chinolin-5,8-chinon,
7-Methylthio-6-(4-chlor-3-trifluormethyl-phenylamino)-chinolin-5,8-chinon,
7-Propylthio-6-(4-chlor-3-trifluormethyl-phenylamino)-chinolin-5,8-chinon,
7-Methylthio-6-(2-trifluormethylthio-phenylamino)-chinolin-5,8-chinon,
7-Ethylthio-6-(3-trifluormethylthio-phenylamino)-chinolin-5,8-chinon,
7-Propylthio-6(2,6-dichlor-4-trifluormethylthio-phenyl-amino)-chinolin-5,8-chinon,
7-Methylthio-6-(2-chlor-4-trifluormethylthio-phenyl-amino)-chinolin-5,8-chinon,
7-Methylthio-6-(3-chlor-4-trifluormethylthio-phenyl-amino)-chinolin-5,8-chinon,
7-Methylthio-6-thiomorpholino-chinolin-5,8-chinon,
7-Methylthio-6-[1-(3-trifluormethyl-4-chlor-phenyl)-piperazinyl]-chinolin-5,8-chinon.
7-Propylthio-6-morpholino-chinolin-5,8-chinon,

Die Verbindungen der Formel (I) sind teilweise bekannt, sie lassen sich nach den oben unter Punkt 3 und 5 angegebenen Verfahren a) bis b) herstellen (vgl. z.B. DE-OS 21 36 037; JP 58 06 76 72; C.-W. Schellhammer et al., Liebigs Ann. Chem. 624 (1959), S. 108-119; O.S. Klimovich et al., Zh. Prikl. Khim. 49 (1976), S. 1823-1826; CA 86 (1976) 29288).

Setzt man bei Verfahren 3a zur Herstellung der neuen 6(7)-aminosubstituierten Chinolin-5,8-chinone und deren Regioisomere als Verbindungen der Formel (II) 6,7-Dichlor-chinolin-5,8-chinon und als Verbindungen der Formel (III) (±)-1-(3,4-Dichlor-phenyl)-ethylamin ein, so läßt sich das Verfahren durch folgendes Reaktionsschema wiedergeben:

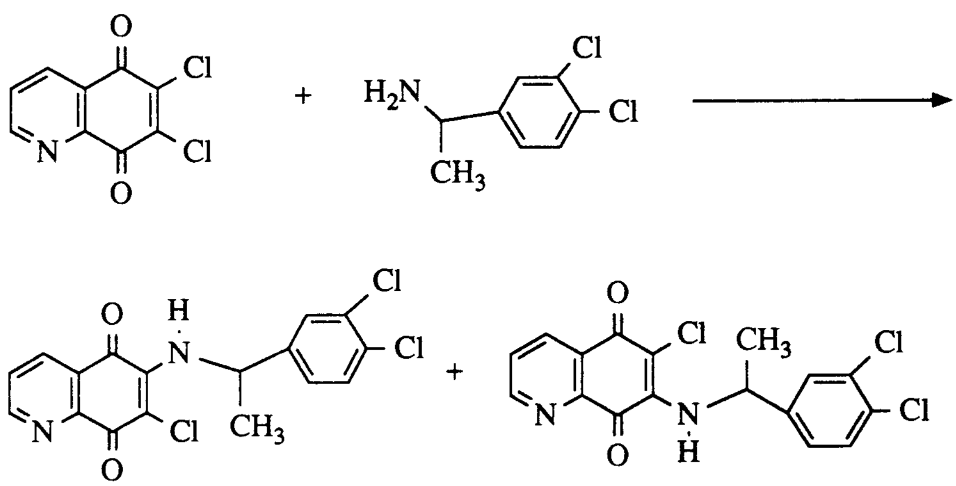

Die zur Durchführung des erfindugnsgemäßen Verfahrens 3a als Ausgangsstoffe benötigten 6,7-Dihalo-chinolin-5,8-chinone sind durch die Formel (II) allgemein definiert. In dieser Formel steht Hal vorzugsweise für diejenigen Halogene, die bereits im Zusammenhang mit der Beschreibung der erfindungsgemäßen Stoffe der Formel (Ia) als bevorzugt für diese Substituenten genannt wurden.

Die als Ausgangsmaterialien verwendeten Verbindungen der Formel (II) sind bekannt (vgl. z.B. T. Urbanski et al., Roczniki Chem. [Ann. Soc. chim. Polonorum] 27 (1953), S. 390-394; CA 49 (1955) 1041; R. Long et al., J. Chem. Soc. [London] (1953), S. 3919; Brit. 856 505) bzw. können nach den dort beschriebenen Verfahren erhalten werden.

Die außerdem für die Durchführung des erfindungsgemäßen Verfahrens 3a an Ausgangsstoffe zu verwendenden Aminoverbindungen der Formel (III) sind allgemein definiert. In dieser Formel (III) haben $R^1$

und $R^2$ die Bedeutung, die bereits im Zusammenhang mit der Beschreibung der erfindungsgemäßen Stoffe der Formel (Ia) als bevorzugt für diese Substituenten genannt wurden. Die Aminoverbindungen der Formel (III) sind in vielen Fällen kommerziell erhältlich oder in an sich bekannter Weise nach der "Leuckart-Wallach-Reaktion" (vgl. z.B. Houben-Weyl, Methoden der Organischen Chemie, Bd. XI/1, 4. Aufl. 1957, G. Thieme Verlag, Stuttgart, S. 648; M.L. Moore in "The Leuckart Reaction" in: Organic Reactions, Bd. 5, 2. Aufl. 1952, New York, John Wiley & Sons, Inc. London: Chapman & Hall, Ltd. (Hrsg. R. Adams), S. 301) darstellbar.

Die Reaktion der Verbindungen der Formel (II) und (III) wird vorzugsweise unter Verwendung von Verdünnungsmitteln durchgeführt. Als Verdünnungsmittel zur Durchführung des erfindungsgemäßen Verfahrens 3a kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglycoldimethylether, Tetrahydrofuran, Dioxan, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitil, Isobutyronitril, Benzonitril, m-Chlorbenzonitril; aliphatische, cycloaliphatische oder aromatische Kohlenwasserstoffe wie Heptan, Hexan, Nonan, Cymol, Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylformamid, N,N-Dimethylformamid, N-Methylpyrrolidon; Ketone wie Aceton, Methylethylketon. Auch Gemische der genannten Lösungs- und Verdünnungsmittel kommen in Betracht.

Bevorzugt sind aliphatische Alkohole.

Das Verfahren 3a wird durchgeführt, indem Verbindungen der Formel (II) und ein Überschuß der Verbindungen der Formel (III) in einem der angegebenen Verdünnungsmittel zusammengegeben und erhitzt werden. Die Reaktionsdauer beträgt ca. 2 bis 10 Stunden. Die Umsetzung erfolgt bei Temperaturen zwischen +20°C und +200°C, bevorzugt zwischen +50°C und +150°C, besonders bevorzugt bei Siedetemperatur des Verdünnungsmittels. Vorzugsweise arbeitet man bei dem Druck, der sich beim Erhitzen auf die erforderliche Reaktionstemperatur unter den Reaktionsbedingungen einstellt.

Zur Durchführung des erfindungsgemäßen Verfahrens setzt man pro Mol an 6,7-Dihalo-chinolin-5,8-chinon der Formel (II) im allgemeinen 1,0 bis 4,0 Mol, vorzugsweise 2,5 bis 3,5 Mol, an Aminoverbindung der Formel (III) ein.

Nach vollendeter Umsetzung wird das Reaktionsgemisch auf 0°C abgekühlt, der ausgefallene Feststoff abfiltriert, gewaschen und getrocknet. Die anfallenden Regioisomerengemische lassen sich in üblicher Weise durch Umkristallisieren reinigen oder chromatographisch in die jeweiligen stellungsisomeren Produkte auftrennen (vgl. auch die Herstellungsbeispiele).

Alternativ kann diese Aminierungsreaktion auch in Gegenwart geeigneter Salze von Metallen der I., III. und VIII. Nebengruppe des Periodensystems der Elemente an einem in situ gebildeten aktivierten Metall-Komplex regioselektiv in 6-Position erfolgen (vgl. z.B.Houben-Weyl, Methoden der Organischen Chemie, Bd. VII/3a, 4. Aufl. 1988, G. Thieme Verlag, Stuttgart, S. 580; Katsuhira Yoshida et al., Bull. Chem. Soc. Jpn. 61 (1988), S. 4335-4340; JP 58 06 76 72). Hierbei sind Chloride des Nickels und Ceriums besonders bevorzugt.

Setzt man bei Verfahren 3b als Verbindungen der Formel (IV) 7-Chlor-6-methoxy-chinolin-5,8-chinon und als Verbindungen der Formel (III) (±)-1-(4-Fluorphenyl)-ethylamin ein, so läßt sich das Verfahren durch folgendes Reaktionsschema beschreiben:

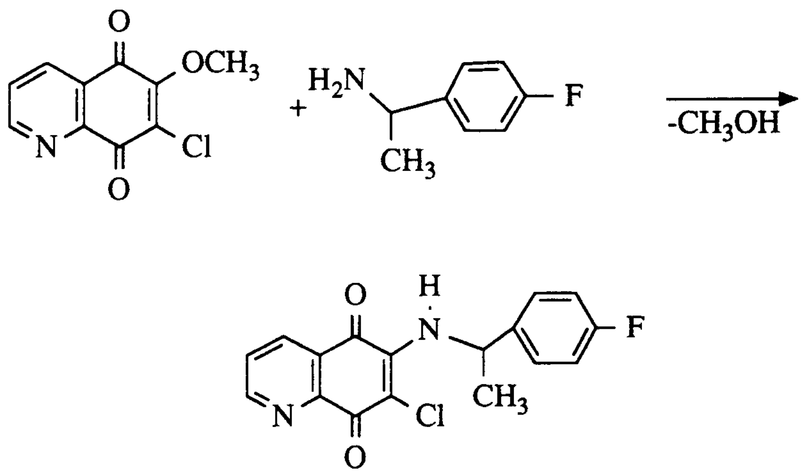


Die Verbindungen der Formel (Ia) werden in diesem Fall regiospezifisch gebildet.

Bevorzugt werden bei Verfahren 3b die Verbindungen der Formel (IV) eingesetzt, bei denen der Rest Hal die bei den Verbindungen der Formel (Ia) genannte bevorzugte und besonders bevorzugte Bedeutung besitzt.

Die zur Durchführung des erfindungsgemäßen Verfahrens 3b als Ausgangsstoffe benötigten Verbindungen der Formel (IV) sind bekannt (vgl. Hsien-Saw Kuo et al., Tai-wan Yao Hsueh Tsa Chih 33 (1981), S. 104-110; CA 97 (1982), 23604; T.K. Liao et al., J. Heterocyclic Chem. 13 (1976), S. 1063-1065).

Die außerdem für die Durchführung des erfindungsgemäßen Verfahrens 3b an Ausgangsstoffe zu verwendenden Amine (III) sind allgemein definiert. Die Amine der Formel (III) sind allgemein bekannte Verbindungen der organischen Chemie.

Das Verfahren 3b wird durchgeführt, indem Verbindungen der Formel (IV) und ein Überschuß der Verbindungen der Formel (III) in einem der angegebenen Verdünnungsmittel zusammengegeben und erhitzt werden. Die Reaktionsdauer beträgt 10 bis 40 Stunden. Die Umsetzung erfolgt bei Temperaturen zwischen +20 ° C und +200 ° C, bevorzugt zwischen +50 ° C und +150 ° C, besonders bevorzugt bei Siedetemperatur des Verdünnungsmittels. Es wird unter Normaldruck gearbeitet.

Zur Durchführung des erfindungsgemäßen Verfahrens setzt man pro Mol an 7-Chlor-6-methoxy-chinolin-5,8-chinon der Formel (IV) im allgemeinen 1,0 bis 4,0 Mol, vorzugsweise 1,5 bis 2,5 Mol, an Aminoverbindung der Formel (III) ein.

Nach vollendeter Umsetzung wird abgekühlt, im Vakuum eingeengt, der ausgefallene Feststoff abfiltriert, gewaschen und getrocknet (vgl. auch die Herstellungsbeispiele).

Setzt man bei Verfahren 5a als Verbindungen der Formel (Ia) 6-[3,5-Bis-(trifluormethyl)phenylamino]-7-chlor-chinolin-5,8-chinon, als Verbindungen der Formel (V) Dinatriumsulfid-Nonahydrat und als Verbindungen der Formel (VII) Dimethylsulfat ein, läßt sich das Verfahren durch folgendes Reaktionsschema beschreiben.

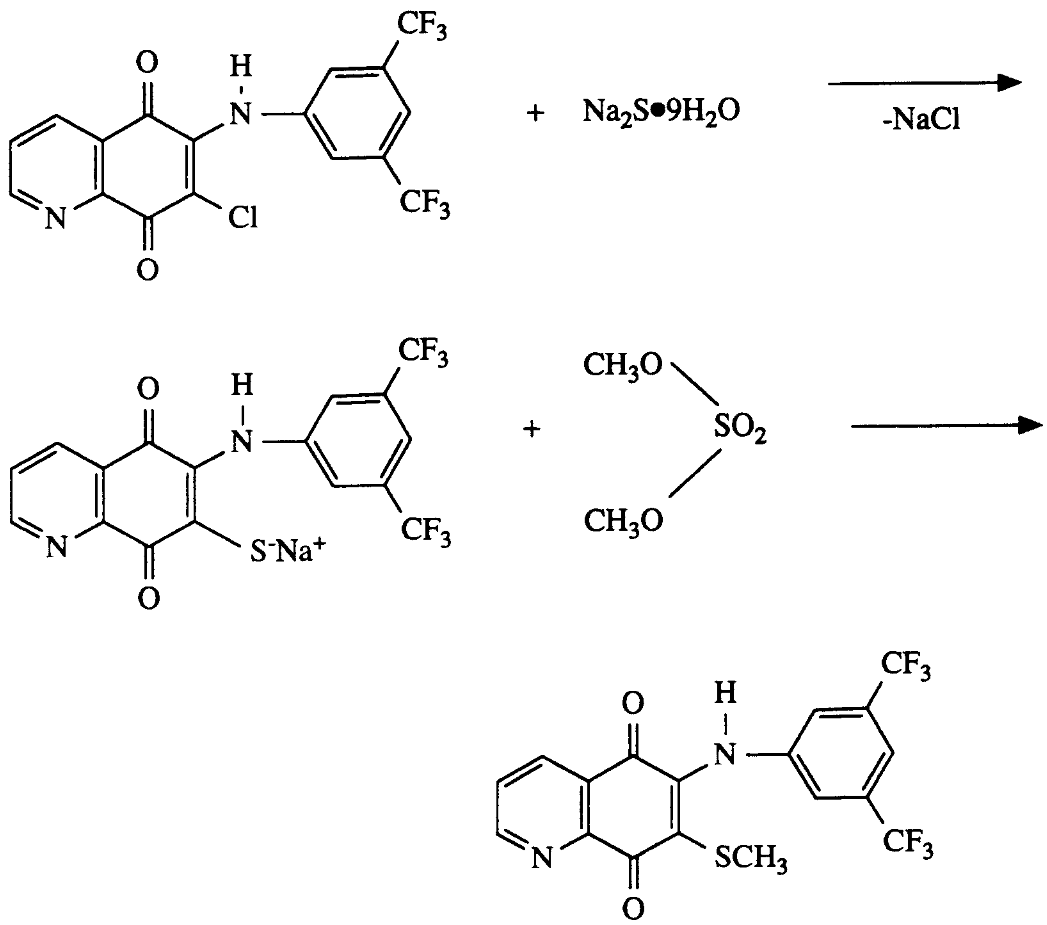


Bevorzugt werden bei Verfahren 5a die Verbindungen der Formel (Ia) eingesetzt bei denen die Reste $R^1$, $R^2$ und Hal die bei den Verbindungen der Formel (I) genannten bevorzugten und besonders bevorzugten Bedeutungen besitzen.

Die außerdem für die Durchführung des erfindungsgemäßen Verfahrens 5a an Ausgangsstoffe zu verwendenden Alkalimetallsulfide der Formel (V) sind allgemein bekannte Verbindungen der anorganischen Chemie.

Die außerdem für die Durchführung des erfindungsgemäßen Verfahrens 5a an Ausgangsstoffe zu verwendenden Alkylierungsmittel der Formel (VII) sind allgemein bekannte Verbindungen der organischen Chemie. In dieser Formel (VII) hat $R^4$ die Bedeutung, die bereits im Zusammenhang mit der Beschreibung der erfindungsgemäßen Stoffe der Formel (I) als bevorzugt für diese Substituenten genannt wurden und E hat die Bedeutung einer elektronenziehenden Abgangsgruppe.

Geeignete Abgangsgruppen E sind z.B. Halogen, wie Chlor, Brom oder Iod, oder ein Rest der Formel $O-SO_2-C_6H_5$, $O-SO_2-C_6H_4-CH_3$ oder $O-SO_2-OR^4$, wobei $R^4$ die oben genannte Bedeutung besitzt.

Die Verwendung von $C_1$-$C_4$-Dialkylsulfaten, insbesondere von Dimethylsulfat oder Diethylsulfat, ist bevorzugt.

Das Verfahren 5a wird durchgeführt, indem Verbindungen der Formel (Ia) und äquimolare Mengen der Verbindungen der Formel (V) in einem der angegebenen Verdünnungsmittel bei Raumtemperatur zusammengegeben werden. Die Ausbildung der in situ gebildeten Alkalimetallsalze der Formel (VI) beträgt ca. 1 bis 30 Minuten.

In einem zweiten Reaktionsschritt erfolgt deren Alkylierung mit Verbindungen der Formel (VII) während einer Reaktionszeit von ca. 10 bis 60 Minuten und einer Reaktionstemperatur zwischen +20 und +200 °C, bevorzugt zwischen +50 und +150 °C, besonders bevorzugt bei Siedetemperatur des Verdünnungsmittels. Es wird unter Normaldruck gearbeitet. Die Aufarbeitung und Isolierung der Reaktionsprodukte wird nach allgemein üblichen Methoden durchgeführt (vgl. auch die Herstellungsbeispiele).

Setzt man bei Verfahren 5b als Verbindungen der Formel (Ia) ein Gemisch der regioisomeren 6-Chlor-7-(4-trifluormethyl-phenylamino)- und 7-Chlor-6-(4-trifluormethyl-phenylamino)-chinolin-5,8-chinone und als Verbindungen der Formel (VIII) Natrium-1-propanthiolat ein, läßt sich das Verfahren durch folgendes

Reaktionsschema beschreiben:

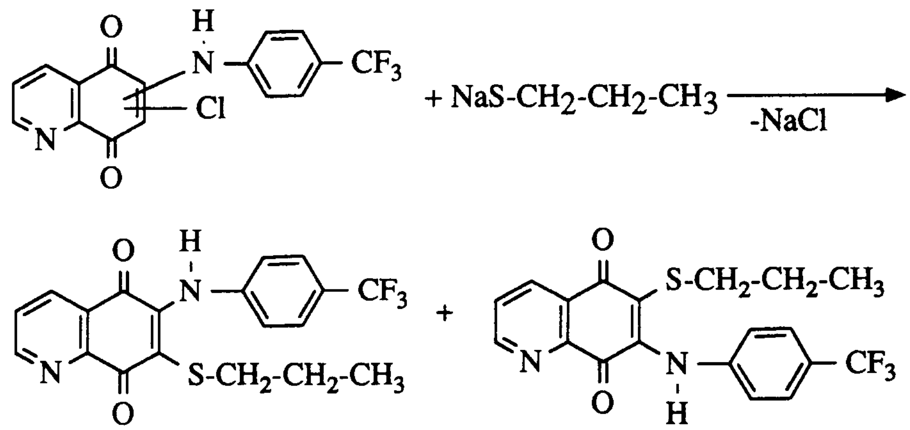


Das Verfahren 5b wird durchgeführt, indem Verbindungen der Formel (Ia) und ein Überschuß der Verbindungen der Formel (VIII) in einem der angegebenen Verdünnungsmittel zusammengegeben und gegebenenfalls erhitzt werden.

Die Reaktionsdauer berägt 10 bis 40 Stunden. Die Reaktion wird bei Temperaturen zwischen 0 ° C und +200 ° C, bevorzugt zwischen +20 ° C und +150 ° C, besonders bevorzugt bei Raumtemperatur, durchgeführt. Es wird bei Normaldruck gearbeitet.

Zur Durchführung des erfindungsgemäßen Verfahrens setzt man pro Mol an Gemisch der regioisomeren 6-Chlor-7-(4-trifluormethyl-phenylamino)- und 7-Chlor-6-(4-trifluormethyl-phenylamino)-chinolin-5,8-chinone der Formel (Ia) im allgemeinen 1,0 bis 3,0 Mol, vorzugsweise 1,0 bis 1,5 Mol, an Alkalimetallsalzen der Thiole der Formel (VIII) ein.

Alternativ kann diese Umsetzung auch mit Thiolen der allgemeinen Formel (VIII), in der M für Wasserstoff steht, in Gegenwart eines basischen Reaktionshilfsmittels durchgeführt werden. Als solche können als Basen alle geeigneten Säurebindemittel eingesetzt werden wie Amine, insbesondere tertiäre Amine sowie Alkali- und Erdalkaliverbindungen. Beispielhaft seien dafür erwähnt die Hydroxide, Oxide und Carbonate des Lithiums, Natriums, Kaliums, Magnesiums, Calciums und Bariums, ferner weitere basische Verbindungen wie Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyltoluidin, N,N-Dimethyl-p-aminopyridin, N-Methyl-pyrrolidin, N-Methyl-piperidin, N-Methyl-imidazol, N-Methyl-pyrrol, N-Methyl-morpholin, N-Methyl-hexamethylenimin, Pyridin, Chinolin, α-Picolin, β-Picolin, Isochinolin, Pyrimidin, Acridin, N,N,N',N'-Tetra-methylendiamin, N,N,N',N'-Tetraethylendiamin, Chinoxalin, N-Propyl-diisopropylamin, N,N'-Dimethylcyclohexylamin, 2,6-Lutidin, 2,4-Luditin, Triethylendiamin, Diazabicyclooctan (DABCO), Diazabicyclononen (DBN) oder Diazabicycloundecen (DBU).

Vorzugsweise verwendet man Alkaliverbindungen, wie beispielsweise Natriumhydroxid, Kaliumhydroxid oder entsprechende Carbonate.

Nach vollendeter Umsetzung wird das Reaktionsgemisch im Vakuum eingeengt (zu ca. 50 %), der Rückstand mit wäßriger Säure versetzt und die Verbindungen der Formel (Ib) in an sich bekannter Weise isoliert, indem sie mit einem geeigneten Lösungsmittel, z.B. Chloroform oder Methylenchlorid, extrahiert werden. Die Verbindungen der Formel (Ia) können anschließend in üblicher Weise, z.B. chromatographisch, gereinigt bzw. in die jeweiligen stellungsisomeren Produkte aufgetrennt werden.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stileasia spp., Cittotaenia spp.,

Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystome spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragoniums spp., Collyriclum spp., Nanophyetus spp., Opisthorchis spp., Clonorchis spp., Metorchis spp., Heterophyes spp., Metagonimus spp..

Aus der Ordnung der Enoplida z.B.: Trichuris spp., Capillaria spp., Trichomosoides spp., Trichinella spp.

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Mashallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Monoliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Hongbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zur Injektion; Halbfeste Zubereitungen;

Formulierungen, bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst

wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele wrden hergestellt, indem Lösungen, die wie bei den Injektions lösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systematisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glykole, Polyethylenglykole, Polypropylenglykole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmonobutylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Siliconöle, Fettsäureester, Triglyceride, Fettalkohole.Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Siliconöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Ketten-

länge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether;
ampholytische Tenside wie Di-Na-N-lauryl-$\beta$-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit, gegebenenfalls unter Zusatz weiterer Hilfsstoffe wie Netzmittel, Farbstoffe, resorptionsfördernde Stoffe, Konservierungsstoffe, Antioxidantien, Lichtschutzmittel, suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen, gegebenenfalls unter Zusatz von Hilfsstoffen, vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 bis 10 Gewichtsprozent.

Zubereitungen, die vor Anwendung verdünnt werden, enthalten den Wirkstoff in Konzentrationen von 0,5 bis 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Beispiel A

In vivo Nematodentest

Trichostrongylus colubriformis/Schaf

Experimentell mit Trichostrongylus colubriformis infizierte Schafe wurden nach Ablauf der Präpatenzzeit

des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 9 | 10 |
| 13 | 10 |
| 15 | 10 |
| 48 | 10 |
| 49 | 10 |

Beispiel B

In vivo Nematodentest

Haemonchus contortus/Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 12 | 10 |
| 17 | 10 |

Herstellungsbeispiele

Beispiel 1

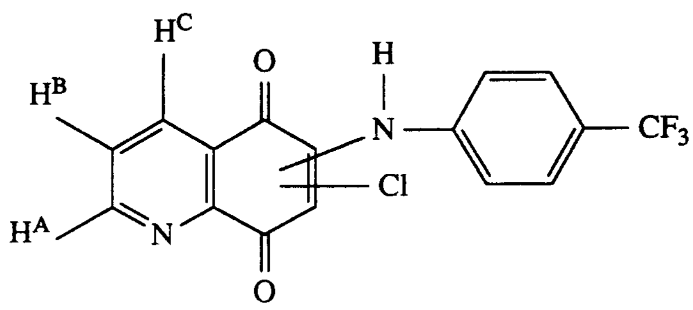

5,0 g (21,9 mMol) 6,7-Dichlor-chinolin-5,8-chinon werden in 35 ml absolutem Ethanol vorgelegt und mit 10,6

g (65,7 mMol) 4-Trifluormethyl-anilin versetzt. Dann wird bis zum vollständigen Umsatz (2,5 Stunden) bei Rückflußtemperatur gerührt und anschließend auf 0°C abgekühlt. Der ausgefallene Feststoff wird abgesaugt und getrocknet. Man erhält 7,7 g Rohprodukt eines Regioisomerengemisches, das über eine Kieselgelsäule (Kieselgel 60 - Merck, Korngröße: 0,040-0,063 mm) mit dem Fließmittel Toluol: Ethanol (15:1) chromatographiert wird.

6-Chlor-7-(4-trifluormethyl-phenylamino)-chinolin-5,8-chinon

Fp.: 238-239°C 1,5g (19,5 % der Theorie)
$^1$H-NMR ($CDCl_3$, $\delta$): 7,16; 7,63 (2d, 4H, arom.; J = 8,4 Hz); 7,73 (dd, 1H, $H^B$; $JH^B,H^A$: 4,8 Hz; $JH^B,H^C$: 7,9 Hz); 7,85 (s, 1H, NH); 8,53 (dd, 1H, $H^C$; $JH^C,H^B$: 7,9 Hz; $JH^C,H^A$: 1,7 Hz); 9,01 (dd, 1H, $H^A$; $JH^A,H^C$: 1,7 Hz; $JH^A,H^B$: 4,8 Hz)ppm.

Beispiel 2

7-Chlor-6-(4-trifluormethyl-phenylamino)-chinolin-5,8-chinon

Fp.: 188-190°C 2,3g (29,9 % der Theorie)
$^1$H-NMR (DMSO-$d_6$, $\delta$): 7,28; 7,65 (2d, 4H, arom.; J = 8,4 Hz); 7,83 (dd, 1H, $H^B$; $JH^B,H^A$: 4,8 Hz; $JH^B,H^C$: 7,9 Hz); 8,39 (dd, 1H, $H^C$; $JH^C,H^B$: 7,9 Hz; $JH^C,H^A$:1,7 Hz); 9,00 (dd, 1H, $H^A$; $JH^A,H^C$: 1,7 Hz; $JH^A,H^B$: 4,8 Hz); 9,57 (s, 1H, NH) ppm

Beispiel 3

7-Chlor-6-(2,3-dimethyl-phenylamino)-chinolin-5,8-chinon

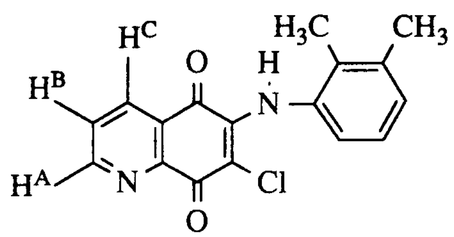

5,0 g (0,022 Mol) 7-Chlor-6-methoxy-chinolin-5,8-chinon werden in 100 ml heißem Ethanol vorgelegt und 5,3 g (0,044 Mol) 2,3-Dimethyl-anilin zugetropft. Dann wird bis zum vollständigen Umsatz (16 Stunden) bei Rückflußtemperatur gerührt und der gesamte Ansatz im Vakuum eingeengt. Der Rückstand wird mit wenig kaltem Ethanol nachgewaschen und aus Ethanol umkristallisiert. Man erhält 3,9 g (56,7 % der Theorie) 7-Chlor-6-(2,3-dimethyl-phenylamino)-chinolin-5,8-chinon.
Fp.: 202-203°C
$^1$H-NMR (DMSO-$d_6$, $\delta$): 2,20; 2,33 (2s, 3H, $CH_3$); 6,98-7,10 (m, 3H, arom.); 7,78 (dd, 1H, $H^B$; $JH^B,H^A$: 4,8 Hz; $JH^B,H^C$: 7,9 Hz); 8,38 (dd,1H, $H^C$; $JH^C,H^B$: 7,9 Hz; $JH^C,H^A$: 1,7 Hz); 8,97 (dd, 1H, $H^A$; $JH^A,H^C$: 1,7 Hz; $JH^A,H^B$: 4,8 Hz); 9,13 (s, 1H, NH) ppm
Analog können die in der nachstehenden Tabelle 1 aufgeführten Verbindungen der Formel (Ia, Hal =Cl) hergestellt werden.

Tabelle 1

Beispiele für die Verbindungen der Formel (Ia)

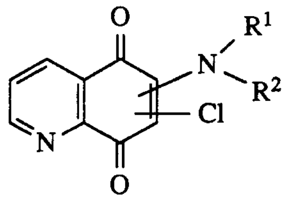 (Ia)

| Beispiel Nr. | Position von Cl | (Position-) 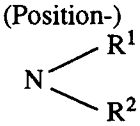 | physikalische Daten |
|---|---|---|---|
| 4 | 6 | (7-) 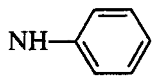 | Fp.: 192°C |
| | 7 | (6-) 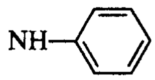 | |
| 5 | 6 | (7-) 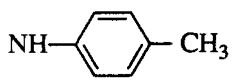 | Fp.: 183°C |
| | 7 | (6-) 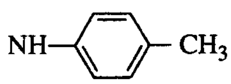 | |
| 6 | 7 | (6-) 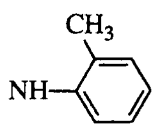 | Fp.: 194-195°C |
| 7 | 7 | (6-) 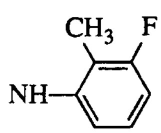 | Fp.: 197-199°C |
| 8 | 7 | (6-) 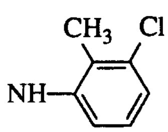 | Fp.: 188-189°C |
| 9 | 7 | (6-) 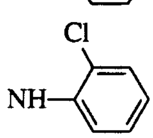 | Fp.: 111-114°C |

Tabelle 1 (Fortsetzung)

| Beispiel Nr. | Position von Cl | (Position-) 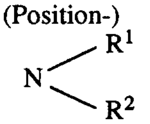 | physikalische Daten |
|---|---|---|---|
| 10 | 6 | (7-) 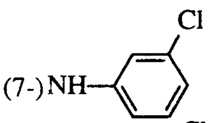 | Fp.: 188°C |
| | 7 | (6-) 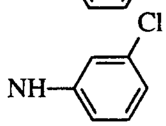 | |
| 11 | 7 | (6-) 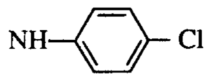 | Fp.: 217-218°C |
| 12 | 6 | (7-) 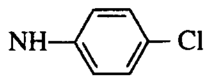 | Fp.: 208°C |
| | 7 | (6-) 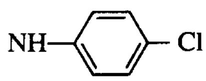 | |
| 13 | 7 | (6-) 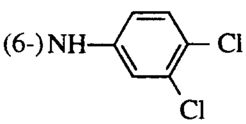 | Fp.: 213-216°C |
| 14 | 7 | (6-) 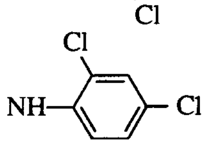 | Fp.: 246-248°C |
| 15 | 7 | (6-) 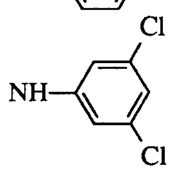 | Fp.: >250°C |
| 16 | 7 | (6-) 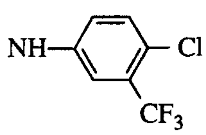 | Fp.: 223-224°C |
| 17 | 6 | (6-) 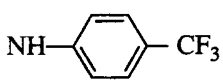 | Fp.: 202°C |
| | 7 | (6-) 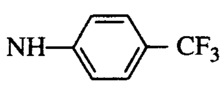 | |

Tabelle 1 (Fortsetzung)

| Beispiel Nr. | Position von Cl | (Position-) 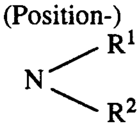 | physikalische Daten |
|---|---|---|---|
| 18 | 7 | (6-) 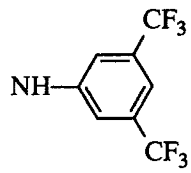 | Fp.: 208-209°C |
| 19 | 7 | (6-) 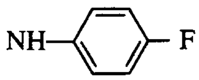 | Fp.: 220-221°C |
| 20 | 7 | (6-) 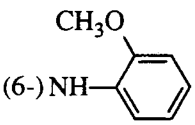 | Fp.: 175-178°C |
| 21 | 6 | (7-) 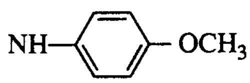 | Fp.: 203°C |
| | 7 | (6-) 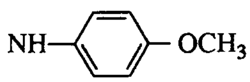 | |
| 22 | 7 | (6-) 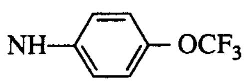 | Fp.: 188-190°C |
| 23 | 7 | (6-) 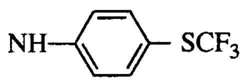 | Fp.: 192-193°C |
| 24 | 7 | (6-) 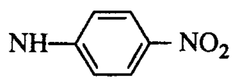 | Fp.: >250°C |
| 25 | 6 | (±)-(7-) 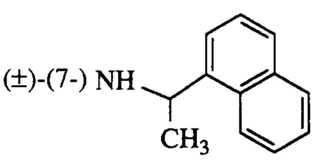 | Fp.: 133°C |
| | 7 | (±)-(6-) 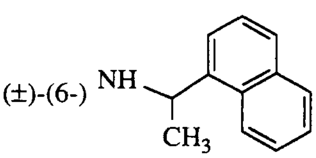 | |

Tabelle 1 (Fortsetzung)

| Beispiel Nr. | Position von Cl | (Position-) 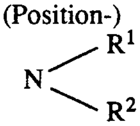 | physikalische Daten |
|---|---|---|---|
| 26 | 6 | (±)-(7-) 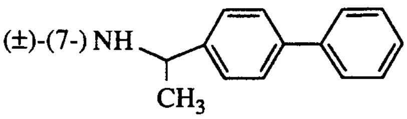 | Fp.: 159°C |
| | 7 | (±)-(6-) 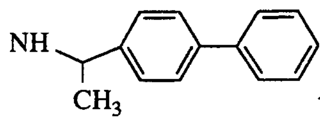 | |
| 27 | 6 | (±)-(7-) 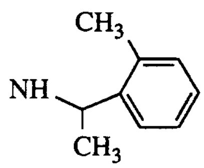 | Fp.: 110°C |
| | 7 | (±)-(6-) 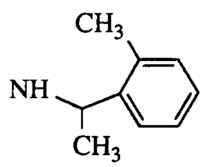 | |
| 28 | 6 | (±)-(7-) 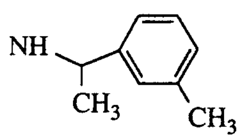 | Fp.: 118°C |
| | 7 | (±)-(6-) 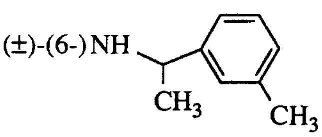 | |
| 29 | 6 | (±)-(7-) 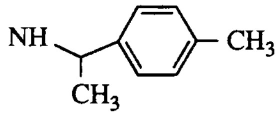 | Fp.: 135°C |
| | 7 | (±)-(6-) 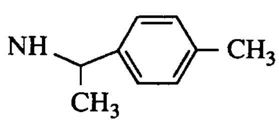 | |

Tabelle 1 (Fortsetzung)

| Beispiel Nr. | Position von Cl | (Position-) 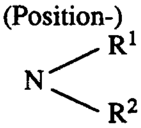 | physikalische Daten |
|---|---|---|---|
| 30 | 6 | (±)-(7-) 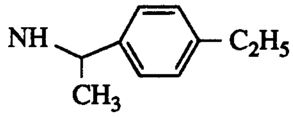 | Fp.: 98°C |
| | 7 | (±)-(6-) 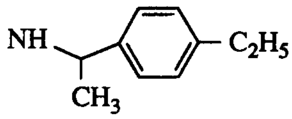 | |
| 31 | 6 | (±)-(7-) 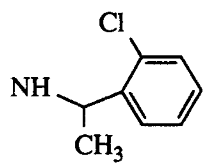 | Fp.: 165°C |
| | 7 | (±)-(6-) 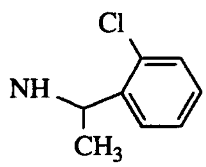 | |
| 32 | 6 | (±)-(7-) 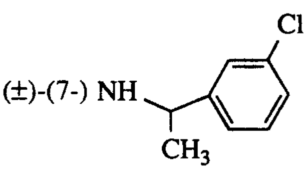 | Fp.: 118°C |
| | 7 | (±)-(6-) 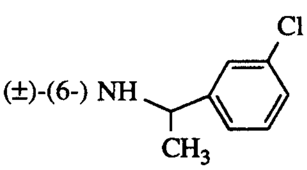 | |
| 33 | 6 | (±)-(7-) 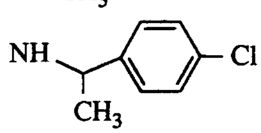 | Fp.: 172°C |
| | 7 | (±)-(6-) 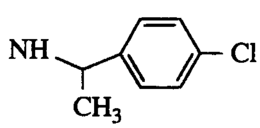 | |

Tabelle 1 (Fortsetzung)

| Beispiel Nr. | Position von Cl | (Position-) 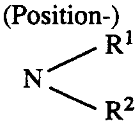 | physikalische Daten |
|---|---|---|---|
| 34 | 6 | (±)-(7-) 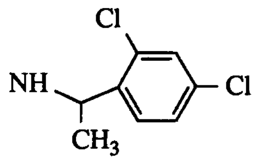 | Fp.: 128°C |
| | 7 | (±)-(6-) 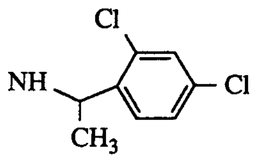 | |
| 35 | 6 | (±)-(7-) 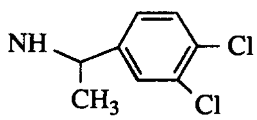 | Fp.: 152°C |
| | 7 | (±)-(6-) 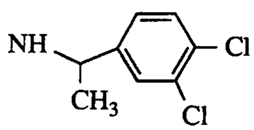 | |
| 36 | 6 | (±)-(7-) 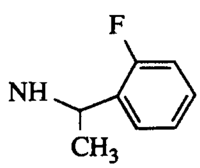 | Fp.: 155°C |
| | 7 | (±)-(6-) 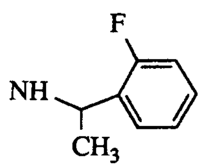 | |
| 37 | 6 | (±)-(7-) 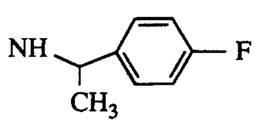 | Fp.: 138°C |
| | 7 | (±)-(6-) 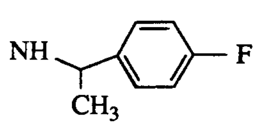 | |

Tabelle 1 (Fortsetzung)

| Beispiel Nr. | Position von Cl | (Position-) 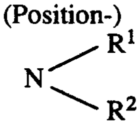 | | physikalische Daten |
|---|---|---|---|---|
| 38 | 6 | (±)-(7-) | 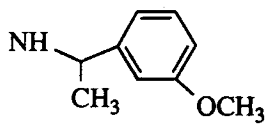 | Fp.: 125°C |
| | 7 | (±)-(6-) | 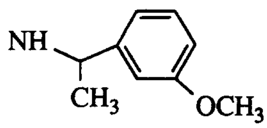 | |
| 39 | 7 | (6-) | NH 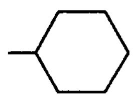 | Fp.: 124-125°C |
| 40 | 7 | (6-) | NH – $(CH_2)_2$- 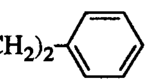 | Fp.: 129-130°C |
| 41 | 7 | (6-) | NH-$(CH_2)_2$-$CF_3$ | Fp.: 160-162°C |
| 42 | 7 | (6-) | NH-$CH(CH_3)_2$ | Fp.: 109-110°C |
| 43 | 7 | (6-) | NH-$CH(CH_3)$-$CF_3$ | Fp.: 180-181°C |
| 44 | 7 | (6-) | NH-$CH(CH_3)$-$C_2H_5$ | Fp.: 77-78°C |
| 45 | 7 | (6-) | NH-$CH_2$-$CH(CH_3)_2$ | Fp.: 102-103°C |
| 46 | 7 | (6-) | NH-$C(CH_3)_3$ | Fp.: 130-131°C |

Beispiel 47

6-[3,5-Bis(trifluormethyl)phenylamino]-7-methylthio-chinolin-5,8-chinon

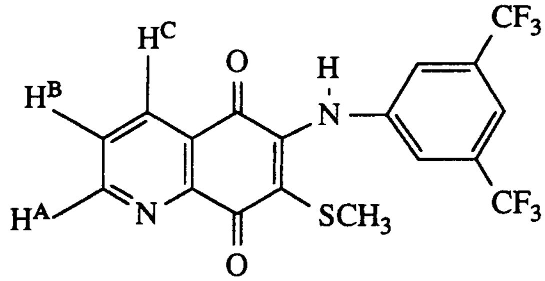

4,6 g (10,9 mMol) 6-[3,5-Bis(trifluormethyl)phenylamino]-7-chlor-chinolin-5,8-chinon werden in 40 ml Ethanol vorgelegt und bei Raumtemperatur mit 2,6 g (10,9 mMol) Dinatriumsulfid Nonahydrat (gelöst in 40 ml Wasser) versetzt. Dabei verfärbt sich die bisher rote Lösung blau-schwarz. Man rührt 5 Minuten bei Raumtemperatur nach, tropft 1,0 g (10,9 mMol) Dimethylsulfat hinzu und erhitzt weitere 15 Minuten am Rückfluß. Nach einstündigem Rühren bei Raumtemperatur wird der gesamte Ansatz im Vakuum eingeengt, der Rückstand mit Wasser verrührt und der abgetrennte Feststoff aus Ethanol umkristallisiert. Man erhält 1,0 g (21,2 % der Theorie) 6-[3,5-Bis(trifluormethyl)phenylamino]-7-methylthio-chinolin-5,8-chinon.
Fp.: 135-137 ° C.
$^1$H-NMR ($CDCl_3$, δ): 2,28 (s, 3H, $-SCH_3$); 7,39 (s, 2H, arom.); 7,63 (s, 1H, arom.); 7,66 (dd, 1H, $H^B$; $JH^A$, $JH^B$: 4,8 Hz; $JH^B$, $H^C$: 7,9 Hz); 7,85 (s, 1H, NH); 8,43 (dd, 1H, $H^C$; $JH^C$, $H^B$: 7,9 Hz; $JH^C$, $H^A$: 1,7 Hz); 9,03 (dd, 1H, $H^A$; $JH^A$, $H^C$: 1,7 Hz; $JH^A$, $H^B$: 4,8 Hz) ppm.

Beispiel 48

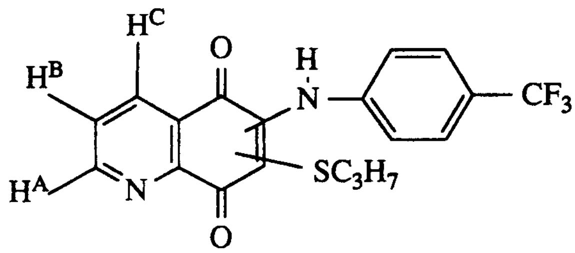

10 g (28,3 mMol) des Gemisches aus 6-Chlor-7-(4-trifluormethyl-phenylamino)- und 7-Chlor-6-(4-trifluormethyl-phenylamino)-chinolin-5,8-chinon werden in 100 ml absolutem Tetrahydrofüran vorgelegt und portionsweise mit 3,4 g (34,6 mMol) Natrium-1-propanthiolat versetzt. Die Mischung wird bis zum vollständigen Umsatz (15 bis 20 Stunden) bei Raumtemperatur gerührt. Danach wird der gesamte Reaktionsansatz im Vakuum eingeengt, der sirupöse Rückstand in 200 ml Chloroform aufgenommen, mit 200 ml 0,5 N Salzsäure ausgeschüttelt und mit Wasser gewaschen. Die organische Phase wird über Nathumsulfat getrocknet und anschließend das Lösungsmittel abdestilliert. Das zurückbleibende Rohprodukt des Regioisomerengemisches kann über eine Kieselgelsäule (Kieselgel 60-Merck, Korngröße: 0,040-0,063 mm) mit dem Fließmittel Toluol:Ethanol (10:1) chromatographiert werden.

6-(1-Propylthio)-7-(4-trifluormethyl-phenylamino)-chinolin-5,8-chinon

Fp.: 175-176 ° C 1,8 g (16,2 % der Theorie)
$^1$H-NMR (DMSO-$d_6$, δ): 0,73 (t, 3H, $-CH_3$; J = 7,3 Hz); 1,31 (m, 2H, $-CH_2-$; J = 7,3 Hz); 2,56 (t, 2H, $-SCH_2-$; J = 7,3 Hz); 7,20; 7,62 (d, 4H; arom. J = 8,4 Hz); 7,82 (dd, 1H, $H^B$; $JH^B$, $H^A$: 4,8 Hz; $JH^B$, $H^C$: 7,9 Hz); 8,36 (dd, 1H, $H^C$; $JH^C$, $H^B$: 7,9 Hz; $JH^C$, $H^A$: 1,7 Hz); 8,96 (dd, 1H, $H^A$; $JH^A$, $H^C$: 1,7 Hz; $JH^A$, $H^B$: 4,8 Hz); 9,49 (s, 1H, NH) ppm.

Beispiel 49

7-(1-Propylthio)-7-(4-trifluormethyl-phenylamino)-chinolin-5,8-chinon

Fp.: 85-86 ° C 1,65g (14,7 % der Theorie)
$^1$H-NMR (DMSO-$d_6$, δ): 0,74 (t, 3H, $-CH_3$; J = 7,3 Hz); 1,32 (m, 2H, $-CH_2-$; J = 7,3 Hz); 2,57 (t, 2H, $-SCH_2-$; J =7,3 Hz); 7,19; 7,61 (2d, 4H; arom. J= 8,4 Hz); 7,79 (dd, 1H, $H^B$; $JH^B$, $H^A$: 4,8 Hz; $JH^B$, $H^C$: 7,9 Hz); 8,38 (dd, 1H, $H^C$; $JH^C$, $H^B$: 7,9 Hz; $JH^C$, $H^A$: 1,7 Hz); 8,98 (dd, 1H, $H^A$; $JH^A$, $H^C$: 1,7 Hz; $JH^A$, $H^B$: 4,8 Hz); 9,39 (s, 1H, NH) ppm.

Analog können die in der nachstehenden Tabelle 2 aufgeführten Verbindungen der Formel (Ib) hergestellt werden.

Tabelle 2

Beispiele für die Verbindungen der Formel (Ib)

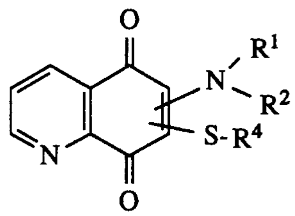 (Ib)

| Beispiel Nr. | (Position-) $S-R^4$ | (Position-) 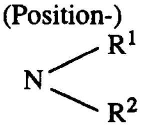 | physikalische Daten |
| --- | --- | --- | --- |
| 50 | (6-) $S-CH_3$ | (7-) 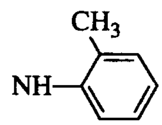 | Fp.: 118°C |
| | (7-) $S-CH_3$ | (6-) 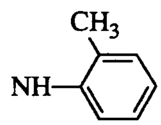 | |
| 51 | (6-) $S-C_3H_7$ | (7-) 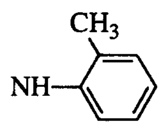 | Fp.: 94-95°C |
| 52 | (7-) $S-C_3H_7$ | (6-) 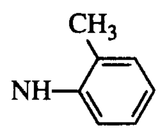 | Fp.: Öl |
| 53 | (6-) $S-CH_3$ | (7-) 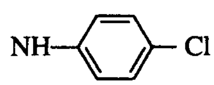 | Fp.: 208-210°C |
| 54 | (7-) $S-CH_3$ | (6-) 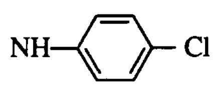 | Fp.: 122-124°C |
| 55 | (6-) $S-C_3H_7$ | (7-) 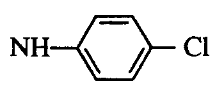 | Fp.: 202-203°C |

Tabelle 2 (Fortsetzung)

| Beispiel Nr. | (Position-) S-$R^4$ | (Position-) 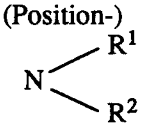 | physikalische Daten |
|---|---|---|---|
| 56 | (7-) S-$C_3H_7$ | (6-) 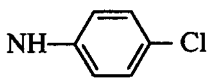 | Fp.: 117-119°C |
| 57 | (7-) S-$CH_3$ | (6-) 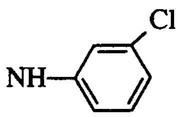 | Fp.: 86-87°C |
| 58 | (7-) S-$C_3H_7$ | (6-) 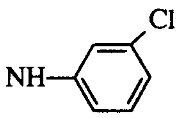 | Fp.: 83-84°C |
| 59 | (7-) S-$CH_3$ | (6-) 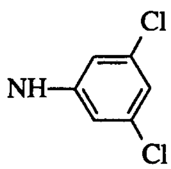 | Fp.: 184-185°C |
| 60 | (7-) S-$C_2H_5$ | (6-) 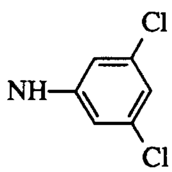 | Fp.: 132-133°C |
| 61 | (6-) S-$CH_3$ | (7-) 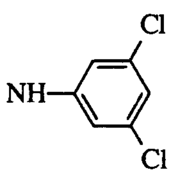 | Fp.: 202-203°C |
| 62 | (7-) S-$C_3H_7$ | (6-) 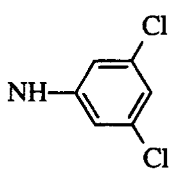 | Fp.: 113-114°C |

Tabelle 2 (Fortsetzung)

| Beispiel Nr. | (Position-) S-$R^4$ | (Position-) 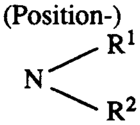 | physikalische Daten |
|---|---|---|---|
| 63 | (6-) S-$CH_3$ | (7-) 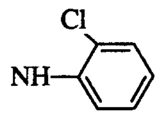 | Fp.: 152°C |
| | (7-) S-$CH_3$ | (6-) 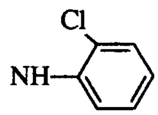 | |
| 64 | (6-) S-$CH_3$ | (7-) 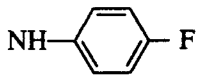 | Fp.: 158-160°C |
| 65 | (7-) S-$CH_3$ | (6-) 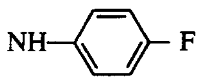 | Fp.: 102-103°C |
| 66 | (7-) S-$C_3H_7$ | (6-) 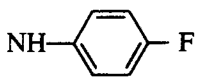 | Fp.: 95- 96°C |
| 67 | (6-) S-$CH_3$ | (7-) 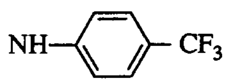 | Fp.: 141-143°C |
| 68 | (7-) S-$CH_3$ | (6-) 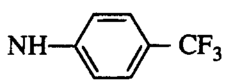 | Fp.: 180-181°C |
| 69 | (6-) S-$CH_3$ | (7-) 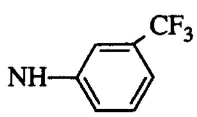 | Fp.: 190-192°C |
| 70 | (7-) S-$CH_3$ | (6-) 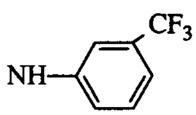 | Fp.: 165-167°C |

Tabelle 2 (Fortsetzung)

| Beispiel Nr. | (Position-) S-$R^4$ | (Position-) 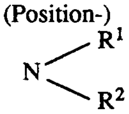 | physikalische Daten |
|---|---|---|---|
| 71 | (6-) S-$C_3H_7$ | (7-) 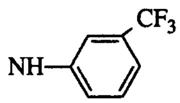 | Fp.: 126-128°C |
| 72 | (7-) S-$C_3H_7$ | (6-) 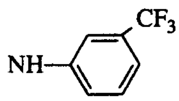 | Fp.: 87- 88°C |
| 73 | (6-) S-$CH_3$ | (7-) 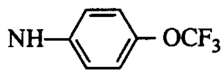 | Fp.: 154-155°C |
| 74 | (7-) S-$CH_3$ | (6-) 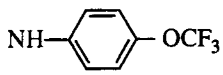 | Fp.: 112- 113°C |
| 75 | (6-) S-$C_3H_7$ | (7-) 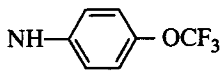 | Fp.: 154-155°C |
| 76 | (7-) S-$C_3H_7$ | (6-) 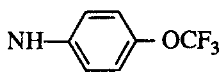 | Fp.: 108-109°C |
| 77 | (6-) S-$CH_3$ | (7-) 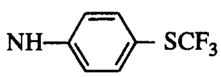 | Fp.: 143°C |
| | (7-) S-$CH_3$ | (6-) 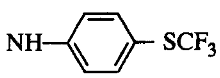 | |

Tabelle 2 (Fortsetzung)

| Beispiel Nr. | (Position-) $S-R^4$ | (Position-) | 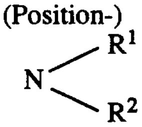 | physikalische Daten |
|---|---|---|---|---|
| 78 | (6-) $S-C_3H_7$ | (7-) | 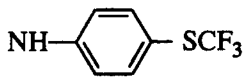 | Fp.: 103°C |
| | (7-) $S-C_3H_7$ | (6-) | 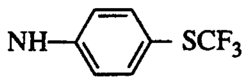 | |
| 79 | (7-) $S-CH_3$ | (6-) | 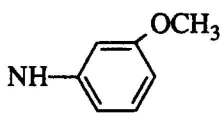 | Fp.: 114-115°C |
| 80 | (7-) $S-C_3H_7$ | (6-) | 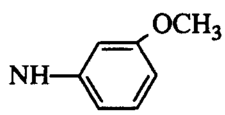 | Fp.: 92°C |
| 81 | (7-) $S-CH_3$ | (6-) | 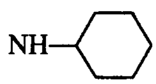 | Fp.: Öl |
| 82 | (6-) $S-CH_3$ | (7-) | 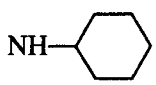 | Fp.: 105-106°C |
| 83 | (6-) $S-C_3H_7$ | (7-) | 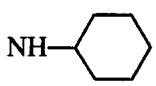 | Fp.: 145-146°C |

Tabelle 2 (Fortsetzung)

| Beispiel Nr. | (Position-) S-$R^4$ | (Position-) | 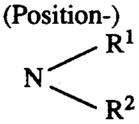 | physikalische Daten |
|---|---|---|---|---|
| 84 | (6-) S-$CH_3$ | (7-) | NH-$CH_2$ 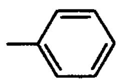 | Fp.: 114°C |
| | (7-) S-$CH_3$ | (6-) | NH-$CH_2$ 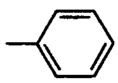 | |
| 85 | (6-) S-$C_3H_7$ | (7-) | NH-$CH_2$ 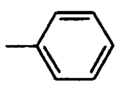 | Fp.: 125-127°C |
| 86 | (7-) S-$CH_3$ | (6-) | NH-$(CH_2)_2$ 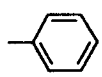 | Fp.: 91- 93°C |
| 87 | (6-) S-$C_3H_7$ | (±)-(7-) | 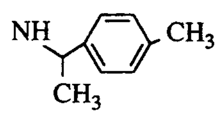 | Fp.: Öl |
| | (7-) S-$C_3H_7$ | (±)-(6-) | 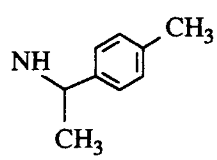 | |
| 88 | (6-) S-$C_3H_7$ | (±)-(7-) | 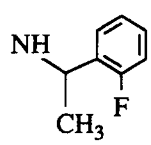 | Fp.: Öl |
| | (7-) S-$C_3H_7$ | (±)-(6-) | 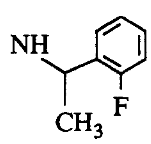 | |

Tabelle 2 (Fortsetzung)

| Beispiel Nr. | (Position-) S-$R^4$ | (Position-) 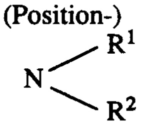 | physikalische Daten |
|---|---|---|---|
| 89 | (7-) S-$CH_3$ | (6-) NH-$(CH_2)_2$-$CF_3$ | Fp.: 142-144°C |
| 90 | (7-) S-$CH_3$ | (6-) NH-CH$(CH_3)_2$ | Fp.: 103-104°C |
| 91 | (7-) S-$CH_3$ | (6-) NH-CH$(CH_3)$-$CF_3$ | Fp.: 102-103°C |
| 92 | (7-) S-$CH_3$ | (6-) NH-CH$(CH_3)$-$C_2H_5$ | Fp.: Öl |
| 93 | (7-) S-$CH_3$ | (6-) NH-$CH_2$-CH$(CH_3)_2$ | Fp.: 81-83°C |
| 94 | (7-) S-$CH_3$ | (6-) NH-C$(CH_3)_3$ | Fp.: 66-68°C |

Beispiele für die Herstellung der Ausgangsprodukte

Beispiel (IV-1)

7-Chlor-6-methoxy-chinolin-5,8-chinon

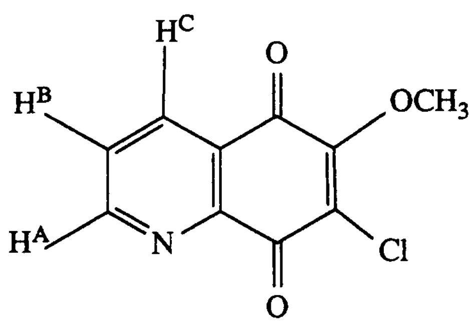

Zu einer auf 0 bis -5 ° C abgekühlten Suspension aus 6,6 g (0,029 Mol) 6,7-Dichlor-chinolin-5,8-chinon in 200 ml Methanol werden portionsweise 2,0 g (0,029 Mol) Kaliummethanolat gegeben. Dabei tritt eine leichte Wärmeentwicklung auf. Anschließend wird noch 2 Stunden bei Raumtemperatur nachgerührt und der gesamte Ansatz im Vakuum eingeengt. Der verbleibende Rückstand wird mit Wasser gewaschen und aus wenig Methanol umkristallisiert. Man erhält 4,5 g (69,4 % der Theorie) 7-Chlor-6-methoxy-chinolin-5,8-chinon.
Fp.: 175-177 ° C
$^1$H-NMR (DMSO-$d_6$, δ): 4,24 (s, 3H, -$OCH_3$); 7,85 (dd, 1H, $H^B$; $JH^B$, $H^A$: 4,8 Hz; $JH^B$, $H^C$: 7,9 Hz); 8,38 (dd, 1H, $H^C$; $JH^C$, $H^B$: 7,9 Hz, $JH^C$, $H^A$: 1,7 Hz); 9,00 (dd, 1H, $H^A$;$JH^A$, $H^C$: 1,7 Hz; $JH^A$, $H^B$: 4,8 Hz) ppm.

## Patentansprüche

1. Verwendung von 6(7)-aminosubstituierten Chinolin-5,8-chinonen der allgemeinen Formel (I)

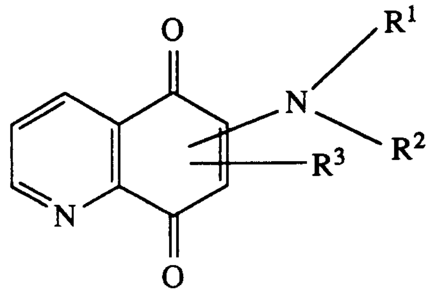

(I),

in welcher

$R^1$ für substituiertes $C_1$-$C_6$-Alkyl, sowie für gegebenenfalls substituiertes Cycloalkylalkyl, Cycloalkyl, Aralkyl oder Aryl steht, wobei als Substituenten genannt seien Halogen, Nitro, Alkyl, Alkylamino, Aminoacyl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl,

$R^2$ für Wasserstoff, Alkyl steht oder $R^1$ und $R^2$ gemeinsam mit dem angrenzenden N-Atom für einen carbocyclischen 5-, 6- oder 7-gliedrigen Ring stehen, der gegebenenfalls auch durch O, N, S unterbrochen sein kann und gegebenenfalls durch $C_{1-4}$-Alkyl substituiert ist,

$R^3$ für Halogen oder -S-$R^4$ steht,

$R^4$ für Alkyl steht,

zur Bekämpfung von Endoparasiten in der Medizin und Tiermedizin.

**2.** 6(7)-aminosubstituierte Chinolin-5,8-chinone der allgemeinen Formel (Ia)

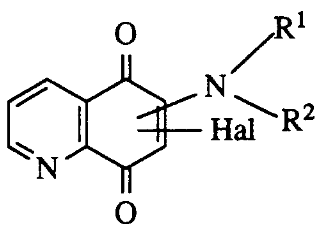

(Ia),

in welcher

$R^1$ für Cycloalkylalkyl oder Aralkyl steht, die gegebenenfalls durch Halogen, Nitro, Alkyl, Alkylamino, Aminoacyl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl substituiert sind,

$R^2$ für Wasserstoff steht,

Hal für Halogen, vorzugsweise Chlor oder Brom, insbesondere Chlor, steht.

**3.** Verfahren zur Herstellung der neuen 6(7)-aminosubstituierten Chinolin-5,8-chinone der Formel (Ia)

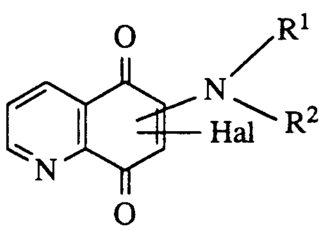

(Ia),

in welcher

$R^1$, $R^2$ und Hal die in Anspruch 2 angegebene Bedeutung besitzen,

dadurch gekennzeichnet, daß man

a) Verbindungen der Formel (II)

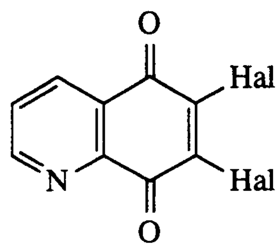

(II),

in welcher

Hal die oben angegebene Bedeutung besitzt,

mit Aminoverbindungen der Formel (III)

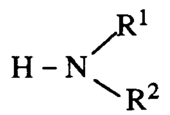

(III),

in welcher

$R^1$ und $R^2$ die oben angegebene Bedeutung haben,

gegebenenfalls in Gegenwart von Metallsalzen und gegebenenfalls in Gegenwart eines Verdünnungsmittels umsetzt, oder indem man

b) Verbindungen der Formel (IV)

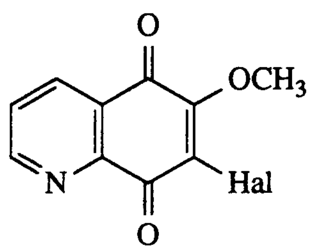

(IV),

in welcher

Hal die oben angegebene Bedeutung besitzt,

mit Aminoverbindungen der Formel (III)

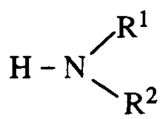

(III),

in welcher

$R^1$ und $R^2$ die oben angegebene Bedeutung haben,

gegebenenfalls in Gegenwart eines Verdünnungsmittels umsetzt.

**4.** 6(7)-aminosubstituierte Chinolin-5,8-chinone der Formel (Ib)

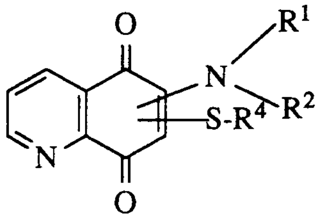

(Ib),

in welcher

$R^1$ für substituiertes $C_1$-$C_6$-Alkyl, sowie für gegebenenfalls substituiertes Cycloalkylalkyl, Cycloalkyl, Aralkyl oder Aryl steht, wobei als Substituenten genannt seien Halogen, Nitro, Alkyl, Alkylamino, Aminoaryl, Aryl, Aryloxy, Arylthio, Aralkyl, Halogenalkyl, Alkoxy, Halogenalkoxy, Alkylthio, Halogenalkylthio, Alkylsulfinyl, Halogenalkylsulfinyl, Alkylsulfonyl oder Halogenalkylsulfonyl,

$R^2$ für Wasserstoff, Alkyl steht oder $R^1$ und $R^2$ gemeinsam mit dem angrenzenden N-Atom für einen carbocyclischen 5-, 6- oder 7-gliedrigen Ring stehen, der gegebenenfalls auch durch O, N, S unterbrochen sein kann und gegebenenfalls durch $C_{1-4}$-Alkyl substituiert ist,

$R^4$ für Alkyl steht.

**5.** Verfahren zur Herstellung der neuen 6(7)-aminosubstituierten Chinolin-5,8-chinone der Formel (Ib)

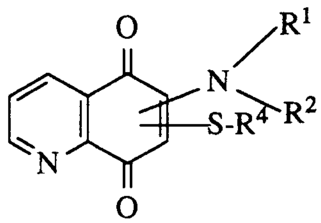

(Ib),

in welcher

$R^1$, $R^2$ und $R^4$ die in Anspruch 4 angegebene Bedeutung besitzen,

dadurch gekennzeichnet, daß man

a) die 6(7)-aminosubstituierten Chinolin-5,8-chinone der Formel (Ia)

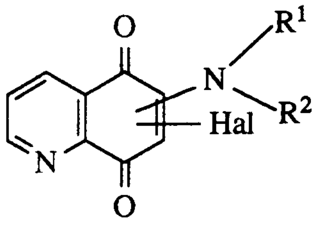

(Ia),

in welcher

$R^1$, $R^2$ und Hal die in Anspruch 2 und 4 angegebene Bedeutung haben,

in einem ersten Reaktionsschritt mit Alkalimetallsulfiden der Formel (V)

$M_2S$ (V),

in welcher

M für ein einwertiges Alkalimetallkation, vorzugsweise Kalium oder Natrium, insbesondere Natrium, steht,

dann in einem zweiten Reaktionsschritt das resultierende Alkalimetallsalz der Formel (VI)

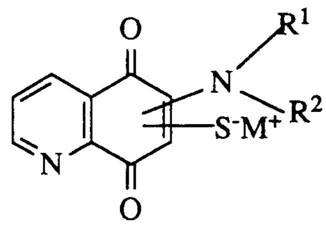

(VI),

in welcher

$R^1$, $R^2$ die oben angegebene Bedeutung besitzen und

M für ein salzartig gebundenes Metallkationenäquivalent steht,

mit Alkylierungsmitteln der Formel (VII)

$R^4$-E (VII),

in welcher

$R^4$ die oben angegebene Bedeutung besitzt und

E für eine elektronenziehende Abgangsgruppe steht,

gegebenenfalls in Gegenwart eines Verdünnungsmittels umsetzt, oder indem man

b) Verbindungen der Formel (Ia)

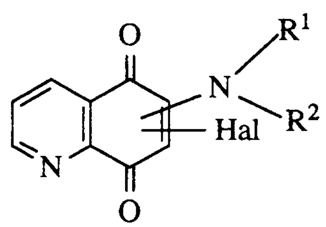

(Ia),

in welcher

$R^1$, $R^2$ und Hal die in Anspruch 2 und 4 angegebene Bedeutung besitzen,

mit Thiolen oder deren Alkalimetallsalzen der Formel (VIII)

$R^4$-$S^-M^+$ (VIII),

in welcher

M für Wasserstoff oder für ein salzartig gebundenes Metallkationenäquivalent steht,

$R^4$ die oben angegebene Bedeutung hat,

in Gegenwart eines Verdünnungsmittels und gegebenenfalls in Gegenwart eines Reaktionshilfsmittels umsetzt.

**6.** Verwendung von 6(7)-aminosubstituierten Chinolin-5,8-chinonen der Formel (I) gemäß Anspruch 1 zur Bekämpfung von Endoparasiten.

**7.** Endoparasitizide Mittel, gekennzeichnet durch einen Gehalt an mindestens einem 6(7)-aminosubstituierten Chinolin-5,8-chinon der Formel (I) gemäß Anspruch 1.

**8.** Verfahren zur Herstellung von endoparasitiziden Mitteln, dadurch gekennzeichnet, daß man 6(7)-aminosubstituierte Chinolin-5,8-chinone der Formel (I) gemäß Anspruch 1 mit Streckmitteln und/oder oberflächenaktiven Mitteln vermischt.

**9.** Verwendung von 6(7)-aminosubstituierten Chinolin-5,8-chinonen der Formel (I) gemäß Anspruch 1 zur

Herstellung von endoparasitiziden Mitteln.

Europäisches Patentamt

# EUROPÄISCHER RECHERCHENBERICHT

Nummer der Anmeldung

EP 92 10 9623

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| | Keine einschlägigen Dokumente gefunden<br>----- | | C07D215/38<br>A01N43/42 |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 14 JULI 1992 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument